# EUROPEAN PATENT SPECIFICATION

(11) **EP 1 780 545 B1**
(45) Date of publication and mention of the grant of the patent: **15.09.2010**
(21) Application number: 05767230.5
(22) Date of filing: 28.07.2005
(51) Int. Cl.: G01N 33/543, G01N 33/53, G01N 33/547, G01N 33/542

(54) **PROBE COMPLEX**
SONDENKOMPLEX
COMPLEXE SONDE

(30) Priority: 30.07.2004 JP 2004223019
(43) Date of publication of application: 02.05.2007
(73) Proprietor: Advanced Life Science Institute, Inc., Wako-shi, Saitama 351-0112 (JP)
(72) Inventor: AOYAGI, Katsumi, Wako-shi, Saitama 3510112 (JP); IIDA, Kumiko, Wako-shi, Saitama 3510112 (JP); MATSUBARA, Naoko, Wako-shi, Saitama 3510112 (JP); ISHIDA, Takehiko, Wako-shi, Saitama 3510112 (JP)
(74) Representative: ter Meer, Nicolaus
(86) International application number: PCT/JP2005/013812
(87) International publication number: WO 2006/011543

(56) References cited:
- JP-A- 2001 013 145
- JP-A- 2002 027 990
- US-A- 4 778 751
- US-A- 5 216 130
- US-B1- 6 252 053
- GREEN S. ET AL: 'PARTIAL PURIFICATION OF A SERUM FACTOR THAT CAUSES NECROSIS OF TUMORS' PROCEEDINGS OF THE NATIONAL ACADEMY OF SCIENCES OF THE UNITED STATES OF AMERICA vol. 73, no. 2, 1976, pages 381 - 385 ISSN: 0027-8424

## Description

### [FIELD OF THE INVENTION]

The present invention relates to a technique for producing probe complexes in which probes and detection markers such as haptens or low molecular peptides are linked to carriers via intermediums. Probe complexes thus produced find a wide range of applications in immunological measurements using immune reactions including enzyme immunoassays and immunohistochemistry.

### [BACKGROUND ART]

Immunohistochemical methods and immunoassays have been used as a method for detecting self-antigens or foreign antigens in organisms utilizing immunological reactions between antigens and antibodies. High specificity and sensitivity of these methods make it possible to detect a small amount of substances in organisms without isolating them.

Immunohistochemical methods are procedures to detect localization of antigens in cells and tissues by means of specific reactions between antigens and antibodies. A broadly used immunohistochemical method by virtue of ABC method is carried out according to the following steps. Tissue sections are prepared from frozen tissues or paraffin fixed tissues. They are subject to blocking with bovine serum albumin and the like in order to prevent non-specific binding. This is reacted with biotinylated antibodies that bind to antigens of interest to form immune complexes between antigens and biotinylated antibodies. To the immune complexes, avidin conjugated enzymes such as horse radish peroxidase (HRP) are added to form biotin-avidin enzyme complexes and antigens of interest are detected by chromogenic or luminescent substrates for the enzymes. Alternatively, detection of antigens of interest can be achieved by adding fluorescent materials including fluorescein or luminescent materials including acridinium esters to avidin.

Immunological measurement methods to detect antigens in biological samples such as serum will be performed according to the following steps. Capturing antibodies bind with antigens to be measured are immobilized to the solid phase such as a microplate. Subsequently, the blocking process is carried out with bovine serum albumin and the like to prevent non-specific adsorption to antibodies and the solid phase. Samples containing antigens to be measured are added to this to bind the antigens of interest with the capturing antibodies. Probe complexes, in which probes such as antibodies recognizing the antigens and haptens such as biotin are linked, will be added to the captured antigens. This results in formation of immune complexes, capturing antibody-antigen-probe complex, on a solid phase such as a microplate. Biotin in the immune complexes is conjugated with HRP-labeled avidin, and chromogenic or luminescent substrates for HRP are added to measure the antigens of interest.

As described above, immunohistochemical methods and immunoassays use probe complexes comprising probes such as antibodies and biotin linked thereto. Also, methods have been developed wherein haptens like dinitrophenyl (DNP), digoxigenin (DIG) or FITC are linked to antibodies instead of biotin to produce probe complexes and enzymes are conjugated to antibodies recognizing these haptens for detection (see, for example, non patent reference 1).

Immunoassays without use of enzymes have been developed wherein probe complexes are prepared by linking, instead of biotin, fluorescent substrates such as fluorescein and luminescent substrates such as acridinium esters to antibodies, then detection is performed using fluorescence or luminescence.

These immunochemical methods and immunoassays are highly sensitive and specific detection methods. However, there are a number of trace substances in organisms that are difficult to be detected by general methods. For example, serum concentration of human gastrin-releasing peptide precursors (proGRP) in normal subjects is approximately 14pg/ml, indicating approximately 1, 000 fold sensitivity is required as compared to carcinnoembryonic antigen (CEA) or α-fetoprotein of which serum concentration in normal subjects are 5-20 ng/mL. Being a foreign antigen, viral level of hepatitis C virus (HCV) in blood is very law, and due to the fact, such sensitivity is required to detect 100-1,000 copies/mL of viral RNA, which is approximately equal to 0.03-0.3pg/mL of core antigens as expressed by protein concentration.

There have been attempts for improvement to increase sensitivity of immunohistochemical methods and immunoassays for detecting trace substances in organisms. Among various factors that may influence the increase in sensitivity of immunohistochemical methods and immunoassays, one factor is to improve functionalities of probe complexes to which probes such as antibodies binding to the antigens described above and biotin are linked. For example, increase in the number of biotin molecules linked to one antibody molecule will lead to increase in the number of molecules including avidin that binds to the biotin and enzymes bound to the avidin, thereby enhancing chromogenic or luminescent signals to cause increase in sensitivity.

However, haptens are linked directly to antibodies to prepare conventional probe complexes that comprise probes such as antibodies and biotin or haptens linked thereto. For example, in one method, amino groups in antibodies and NHS esters introduced into biotin are reacted to link biotin to antibodies via covalent binding. In this method, studies of reaction conditions will increase the number of biotin molecules linked to one antibody molecule. However, the number of amino groups contained in antibodies and the number of biotin that can be linked to the antibodies are both limited. In addition, biotin linked to amino groups in the vicinity of antigen determinants of antibodies may cause adverse effects on association of antibodies with their antigens due to steric hindrance.

Moreover, when haptens to be linked are hydrophobic substances, probe complexes as a whole will have enhanced hydrophobicity through the binding of many hydrophobic haptens to antibodies. In conducting immunohistochemical methods and immunoassays, strong hydrophobicity of probe complexes will lead to non-specific binding of the complexes to tissues or plates due to hydrophobic bonding and resultant increase in background prevents achievement of sufficient sensitivity.
Non-patent reference 1: Harlow E, and Lane, D. "Antibodies: A LABORATORY MANUAL" (U.S.), Cold Spring Harbor Laboratory, 1988, p.340-341.

US 5,216,130 discloses a macro-molecular complex for target-localizing comprising a bio-compatible core, a spacer arm, a specific targeting molecule and a process for producing said complex. The complexes might be used to detect sites of clinical interest using non-invasive external imaging devices.

US 4,778,751 relates to a method of measuring circulating antigens or antibodies by using a ligand labeled specific antigen or ligand labeled specific antibody, chemically attached to a soluble matrix or backbone, a differently labeled anti-antigen or anti-antibody and a solid phase anti-ligand directed at the ligand, attached to the specific antigen or specific antibody.

US 6,252,053 relates to an enzyme-antibody complex, where an enzyme, into which a maleimide group or thiol group is introduced, is covalently conjugated to a carrier. The resulting complex is useful as an enzyme-labeled antibody, particularly in enzyme immunoassay.

### [SUMMARY OF THE INVENTION]

Accordingly, it is an objective of the present invention to provide a soluble highly sensitive probe complex with high functional capability ensuring usability in immunoassays of high sensitivity.

To address issues described above, the inventors have now found a highly sensitive probe complex according to claim 1, which can be produced by directly linking hydrophilic intermediums to a carrier and directly linking detection markers such as biotin, haptens, radioisotopes or low-molecular-weight peptides and antibodies to the intermediums, and thereby completed the present invention. By virtue of the linking of hydrophilic intermediums to a carrier, an increased number of the detection markers can be linked and the probe complex as a whole becomes hydrophilic, so that a probe complex with high functional capability has been completed which would not induce nonspecific reactions.

### [BRIEF DESCRIPTION OF FIGURE]

### [Figure 1]

A schematic view of probe complexes according to the present invention.

### [ADVANTAGEOUS EFFECT OF THE INVENTION]

The probe complexes according to the present invention are probe complexes improved to detect antigens and proteins endogenous to organisms. Use of these probe complexes enables measurement of antigens and proteins by means of immunohistochemical methods and immunoassays, which could not be measured previously.

In addition, since hydrophilicity of intermediums increases that of probe complexes, overall hydrophobicity will be reduced even if detection markers such as haptens, biotin, radioisotopes and low-molecular-weight peptides are hydrophobic. Reduction in non-specific reactions through hydrophobic bonds in immune reactions will thus be expected.

### [DESCRIPTION OF THE INVENTION]

In the present invention, hydrophilic substances as intermediums are linked to carriers and probes and detection markers such as biotin, haptens, radioisotopes, low-molecular-weight peptides and lectins are linked directly to the hydrophilic substances. Detection markers used herein are labeled substances to use the probe complexes according to the present invention in immune reactions.

In the present invention, there is no limitation on carriers as long as their molecular weight ranges 20,000-4,000,000. However, in order to enhance sensitivity, carriers having molecular weight above a certain level are desirable so that probes like antibodies and haptens can bind to them in large amounts. Examples of carriers include polysaccharide and high-molecular-weight proteins and peptide polymers, and they have suitably molecular weight of 20,000-20,000,000, preferably 20,000-4,000,000, more preferably 70,000-2,000,000. When polysaccharides and peptide polymers are used, carriers having more side chains can bind more intermediums as compared to those having same molecular weight, but less side chains.

Polysaccharide carriers as used for the present invention include, but not limited to, dextran, aminodextran, ficoll, dextrin, agarose, various celluloses, chitin, water soluble chitosan and soluble starch. High molecular weight proteinaceous carriers in the present invention include, but not limited to, β-galactosidase, thyroglobulin and hemocyanin and the like. Peptide polymer carriers available in the present invention are various peptide polymers including polylysine.

Various water soluble substances with a molecular weight not less than 2,000 can be used as intermediums in the present invention. Intermediums, for example, include proteins such as bovine serum albumin, human serum albumin, transferrin, ribonuclease, casein, hemoglobin, and ovalbumin as well as avidin soluble chitosan. Peptide polymers like polylysine are also available.

The probes may be any proteins that can bind to substances to be measured, such as antibodies (monoclonal antibodies and polyclonal antibodies) and fragments thereof (F(ab')2, Fab', Fab, F(abc'), Fabc' and the like) and various receptors. Various avidins (avidin D, streptavidin and the like), protein A, protein G, protein L, various lectins (concanavalin A, lentil lectin, Phaseolus Vulgaris lectin and the like) and nucleic acid bound probe analytes and the like may also be used.

Any antibodies are available so long as they bind antigens of interest or analytes. Treatment of antibodies with proteases such as pepsin and papain can produce fragments like F(ab')₂ and Fab. Heavy chains (H chains) of antibodies are generally linked with each other via S-S bonding, and the bonding will be cleaved by reducing agents. Reducing agents include cysteamine and mercaptoethanol, and F(ab')2 also may be cleaved into Fab' by reducing agents, thereby generating new thiol (SH) groups. Such antibody fragments (F(ab')₂, Fab' , Fab, F(abc'), Fabc' and the like) are available in the present invention.

To the intermediums according to the present invention, substances such as haptens, low-molecular-weight peptides and lectins can be linked as detection markers, that are available for detection of signals produced by immune reactions. Detection markers are labeled substances to permit the use of probe complexes in immunological measurements. Contemplated as such detection markers are biotin or haptens. Haptens include dinitrophenyl (DNP), digoxigenin (DIG) and FITC and the like. When biotin is linked to probe complexes, for example, avidin with affinity to biotin is labeled with enzymes such as HRP, fluorescent substances such as fluorescein or luminescent materials such as acridinium esters, is reacted with the probe complexes and signals can be detected by color development, fluorescence or luminescence and the like.

Also, fluorescent materials and luminescent materials such as fluorescein isothiocyanate (FITC) and acridinium esters can be directly linked to the intermediums as haptens. Again, signals can be detected by fluorescence or luminescence.

In addition, direct linking of radioisotopes to intermediums and linking of radioisotope-labeled haptens to intermediums are allowed. In this case, signals can be detected by their radioactivity.

Furthermore, DIG and DNP can be linked as haptens. Here, signals generated by reacting DIG- or DNP-binding antibodies labeled with enzymes, fluorescent materials or luminescent materials are detected. In this instance, any materials are available as detection markers for immunological measurement systems so long as antibodies against haptens are obtainable.

Besides haptens, low-molecular-weight peptides are available as detection markers. The low-molecular-weight peptides are linked to intermediums of probe complexes, and labeling of antibodies against the peptides with enzymes, fluorescent materials or luminescent materials enables signal detection. These peptides may contain sugar chains or lipids.

In case of detecting signals of immunological reactions by means of antibodies against detection markers, materials other than haptens and low-molecular-weight peptides are also available provided that the detection markers are antigenic materials against which antibodies can be raised and can be linked to intermediums.

Substrates for luminescent or chromogenic enzymes are also available as detection markers by being linked to intermediums, because reaction with enzymes for their substrates allows signal detection.

Detection markers to be linked to the probe complexes according to the present invention have desirably molecular weight not more than 10, 000. This is because those markers with higher molecular weight may fail to contribute to improvement in sensitivity of immunoassays due to limited numbers of molecules that are linked to intermediums.

In addition, various lectins (concanavalin A, lentil lectin, Phaseolus Vulgaris lectin and the like) are also available as detection markers.

We now make a brief illustration on a manufacturing method of probe complexes without any limitation.

In the present invention, complexes in which carriers and intermediums are linked are produced. For example, when they are linked to sugar-chain-bearing dextran or ficoll and the like serving as carriers and to proteins such as BSA and casein serving as intermediums, dextran or ficoll are first reacted with sodium periodate to generate aldehyde groups. The aldehyde groups react with amino groups in proteins like BSA, resulting in formation of complexes comprising carriers and intermediums.

The complexes have not only increased hydrophilicity as compared with those with carriers alone but also have increased areas available for linking with probes and haptens and the like.

Probes and haptens as detection markers are then linked to intermediums. In order to control amounts of linked probes and haptens, functional groups are used for each linking are desirably different from each other. For example, if biotin as haptens and Fab' as probes are linked to BSA (intermediums), the following method will be contemplated.

NHS esters of Sulfo-NHS-LC-Biotin, in which NHS (N-hydroxysuccinimide) esters are linked to biotin, are bound to amino groups in BSA. At the same time, linkers are introduced into BSA to link Fab' and BSA. S-S bonds within the BSA are reduced with cysteamine hydrochloride, DTT (Dithiothreitol) and the like to generate thiol groups. The thiol groups are reacted with 1,2-bismaleimide as linkers to introduce maleimide groups. Reaction between the maleimide groups and thiol groups of Fab can give probe complexes comprising carriers, intermediums, detection markers and probes.

Detection markers include various haptens, radioisotopes and peptides. When peptides are linked to the intermediums, cysteines are introduced to peptide terminals, for example, and the thiol groups and amino groups in the intermediums such as BSA may be used for linking. Succinimidyl ' groups and the like may also be introduced into the intermediums using linkers to react them with amino groups in peptides.

Covalent bonding is available for linkage of carriers as described above to intermediums, linkage of intermediums to detection markers and linkage of intermediums to probes. Various functional groups can be used for covalent bonding. For example, the covalent bonding between aldehyde groups and amino groups, hydrazine groups and aldehyde groups, maleimide groups and thiol groups, succinimidyl groups and amino groups, vinyl groups and hydroxyl groups, vinyl groups and thiol groups, but not limited to linkages between these functional groups, are available. If carriers, intermediums, detection markers and probes lack appropriate functional groups, linkage will be achieved by introducing linker molecules having functional groups.

Any linkers will be acceptable provided they have two or more functional groups which may be either different or same.

The resulting probe complexes which comprise carriers, intermediums, probes and haptens or low-molecular-weight peptides as detection markers have find use in immunological reactions such as immunohistochemistry and immunoassays. In case biotin is used, detection can be achieved by linking antigens to probe complexes, then reacting avidin-HRP and adding colorimetric substrates or luminescent substrates. Instead of HRP, avidin bound to enzymes such as alkaline phosphatase, β-galactosidase, glucose oxidase or luciferase linked thereto is available for detection.

Alternatively, acridinium esters and derivatives thereof can be linked to probe complexes. Acridinium esters can react with amino groups in BSA etc. because they have NHS esters. Acridinium esters are available for chemiluminescent immunoassays and can be used as a high sensitive measurement system.

When DNP, DIG, FITC or low-molecular-weight peptides are used as detection markers, they can be linked to intermediums via covalent bonding. Enzymes such as HRP can be linked to antibodies that can bind to these materials specifically, and the detection can be carried out by using color development or luminescence.

### [EXAMPLES]

We now describe Examples regarding a manufacturing method of probe complexes and their performance in order to illustrate the present invention in more detail and without limitation.

### (Example 1)

Preparation of biotin-anti HCV core antigen monoclonal antibody complex with Ficoll 400 as a carrier

44mg of Ficoll 400 (Amersham Bioscienses) was weighted and dissolved in 0.8mL of 0.1M phosphate buffer (pH 7.0) and 0.4mL of sodium periodate solution was added and mixed. After the incubation for two hours at room temperature, excess sodium periodate was removed by gel filtration (Sephadex G25, Amersham Bioscienses), Bovine Serum Albumin (BSA) solution was added and reacted for 3 hours at room temperature, and BSA was introduced into Ficoll. To stabilize the reaction product, Dimethylamine Borate (DMAB; Seikagaku Corporation) was added and mixed to react for 1 hour at room temperature, then Tris solution was added to block unreacted aldehyde groups on Ficoll. After overnight reaction at room temperature, the reaction product was purified by gel filtration (Sephacryl S300, 1.6*30) and absorbance at 280nm was measured to calculate the concentration of carrier BSA conjugates. 1mg of the carrier BSA conjugates were reduced with cysteamine hydrochloride, excess cysteamine hydrochloride was removed by gel filtration (Sephadex G25, Amersham Biosciences), 1,2-bismaleimide and Sulfo-NHS-LC Biotin (Pierce #21335) aqueous solution which were dissolved in dimethylformamide were added and mixed and then the mixture was incubated for 1.5 hours reaction at room temperature, and maleimide groups and biotin were introduced in the carrier BSA conjugates. Excess 1,2 -bismaleimide and Sulfo-NHS-LC Biotin were removed by gel filtration (Sephadex G25, Amersham Biosciences). To the solution of F(ab)'₂ of anti-HCV core antigen monoclonal antibodies in 0.1M phosphate buffer (pH 6.0) (a mixture of equal amount of C11-14 F (ab)'₂ and C11-9 F (ab)'₂) was added 1/10 volume of 0.15M cysteamine hydrochloride, the mixture was incubated for 1.5 hours at 37C°, excess cysteamine hydrochloride was removed by gel filtration (Sephadex G25, Amersham Biosciences) to obtain anti-HCV core antigen monoclonal antibody Fab'. This Fab' and the maleimide and biotin conjugated carrier-BSA were mixed, incubated overnight at 4C°, gel filtration (Sephacryl S300, 1.6x30) was performed to remove free Fab' . In this case, the carrier BSA-Fab' complex according to the present invention appeared close to void fractions, and in view of exclusion limit of Sephacryl S300 being molecular weight of 1.5 million, the molecular weight of the complex was estimated to be several hundred thousand or more. The absorbance at 280nm of the carrier BSA-Fab' complexes were prepared in this way was measured, and the concentration was calculated assuming that the absorption is equal to that of the antibody.

### (Example 2)

Preparation of biotinylated anti-HCV core antigen monoclonal antibodies by a conventional method
Preparation of the biotinylated antibodies was performed according to the method described in a document attached to Sulfo-NHS-LC-Biotin (Pierce #21335). The Sulfo-NHS-LC-Biotin was mixed to anti-HCV core antigen monoclonal antibody in PBS (mixture of an equal amount of C11-14 IgG and Cll-9 IgG), and was incubated for one hour at room temperature, excess Sulfo-NHS-LC-Biotin was then removed by gel filtration (Sephadex G25, Amersham Biosciences) . The absorbance at 280nm of biotinylated anti-HCV core antigen monoclonal antibodies was measured to calculate antibody concentration.

### (Example 3)

Comparison of the biotin-anti-HCV core antigen monoclonal antibody complexes were prepared in Example 1 with the biotinylated anti-HCV core antigen monoclonal antibodies were prepared by a conventional method in Example 2.

Anti-HCV core antigen monoclonal antibodies were adjusted to be 4µg/mL with 0.1M acetate/0.1M phosphate buffer (pH 4.8), 250µl was added to each well of 96-well microplate, and each well was incubated overnight at 4 C°. After washing with PBS, 350µl of 0. 5% casein was added to each well, and each well was incubated for three hours at room temperature. Recombinant HCV core antigens (c11) prepared to concentrations of 0 fmol/L, 148 fmol/L, 444 fmol/L, 1333 fmol/L, 4000 fmol/L, 12000 fmol/L and 36000 fmol/L were added as samples, and each well as incubated for one hour at room temperature with stirring. After washing six times with 10 mM phosphate buffer pH 7.3 containing 0.05% Tween 20 (washing solution). 200µl each of the biotin-anti-HCV core antigen monoclonal antibody complexes were prepared in Example 1 and the biotinylated anti HCV core antigen monoclonal antibodies which were prepared in Example 2 by conventional methods at the concentration of 1µg/mL as secondary antibodies were added to each well, and each well was incubated for one hour at room temperature. After washing six times with the washing solution, 200µL of 5,000-fold diluted HRP-linked-avidin solution was added to each well, and each well was incubated for 30 minutes at room temperature. Subsequently, after washing with the washing solution six times, 200µL of substrate solution (ortho-phenylenediamine - hydrogen peroxide mixture) was added to each well, each well was incubated for 30 minutes at room temperature, and 50µL of 5N sulfuric acid was added to terminate the reaction. Absorbance at 492nm (reference wavelength 600nm) was measured on a microplate reader (MPRA4i, TOSOH). Table 1 shows values obtained by subtracting absorbance values at 0 fmol/L from those of wells to which each concentration of the recombinant HCV core antigens (c11) were added.

**[Table 1]**

| Core antigen concentration | biotinylated antibody prepared by a conventional method | biotin-antibody complex prepared according to Example 1 |
|---|---|---|
| 36000 fmol/L | 2.083 | 2.983 |
| 12000 fmol/L | 0.844 | 2.353 |
| 4000 fmol/L | 0.330 | 1.213 |
| 1333 fmol/L | 0.107 | 0.509 |
| 444 fmol/L | 0.030 | 0.184 |
| 148 fmol/L | 0.002 | 0.052 |
| (value at 0 fmol/L: | 0.024 | 0.017) |

As shown in Table 1, we could not find any difference between 444 fmol/L of core antigens and 0 fmol/L when biotinylated antibodies by conventional methods were used, whereas the biotin-antibody complexes were prepared in Example 1 clearly allowed sufficient detection of 444 fmol/L of core antigens and even 148 fmol/L of core antigens which were further diluted 3 fold. When absorbance was compared, the present biotin-antibody complexes exhibited approximately five fold higher absorbance at 1333 fmol/L and approximately six fold higher absorbance at 444 fmol/L than that obtained by a conventional method.

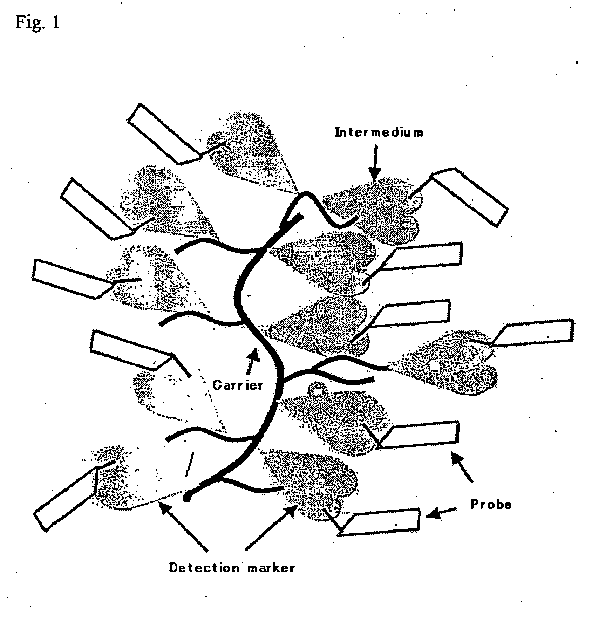

## Claims

1. A probe complex in which proteins or peptide polymers with a molecular weight of 2,000 or more as a hydrophilic intermedium is directly covalently linked to a carrier with a molecular weight of 20,000 - 4,000,000, and a probe and a labeled substance(s) or a substance(s) capable of binding to a labeled substance as a detection marker are directly covalently linked to the intermedium.

2. A probe complex according to claim 1 wherein the detection marker has a molecular weight of 10,000 or less.

3. The probe complex according to claim 1 or 2 wherein the detection markers are biotins.

4. The probe complex according to claim 1 or 2 wherein the detection markers are haptens.

5. The probe complex according to claim 1 or 2 wherein the detection markers are haptens, wherein the hapten is acridinium ester.

6. The probe complex according to claim 1 or 2 wherein the detection markers are fluorescent substances or luminescent substances.

7. The probe complex according to claim 1 or 2 wherein the detection markers have radioisotopes.

8. The probe complex according to claim 1 or 2. wherein the detection marker has fluorescent materials and luminescent materials, such as fluorescein isothiocyanate (FITC) and acridinium ester.

## Patentansprüche

1. Sondenkomplex, bei dem Proteine oder Peptidpolymere mit einem Molekulargewicht von 2.000 oder mehr als ein hydrophiles Zwischenmedium direkt kovalent an einen Träger mit einem Molekulargewicht von 20.000-4.000.000 gebunden sind, und eine Sonde und eine markierte Substanz(en) oder eine Substanz(en), welche fähig ist, an eine markierte Substanz als ein Detektionsmarker zu binden, direkt kovalent an das Zwischenmedium gebunden sind.

2. Sondenkomplex nach Anspruch 1, wobei der Detektionsmarker ein Molekulargewicht von 10.000 oder weniger aufweist.

3. Sondenkomplex nach Anspruch 1 oder 2, wobei die Detektionsmarker Biotine sind.

4. Sondenkomplex nach Anspruch 1 oder 2, wobei die Detektionsmarker Haptene sind.

5. Sondenkomplex nach Anspruch 1 oder 2, wobei die Detektionsmarker Haptene sind, wobei das Hapten Acridiniumester ist.

6. Sondenkomplex nach Anspruch 1 oder 2, wobei die Detektionsmarker fluoreszierende Substanzen oder lumineszierende Substanzen sind.

7. Sondenkomplex nach Anspruch 1 oder 2, wobei die Detektionsmarker Radioisotope aufweisen.

8. Sondenkomplex nach Anspruch 1 oder 2, wobei der Detektionsmarker fluoreszierende Materialien und lumineszierende Materialien aufweist, wie Fluoresceinisothiocyanat (FITC) und Acridiniumester.

## Revendications

1. Complexe à sondes dans lequel des protéines ou des polymères peptidiques avec un poids moléculaire de 2000 ou plus comme milieu intermédiaire hydrophile sont directement reliés de manière covalente à un porteur avec un poids moléculaire de 20 000 - 4 000 000, et une sonde et une/des substance(s) marquée(s) ou une/des substance(s) capable(s) de se lier à une substance marquée comme marqueur de détection sont directement reliées de manière covalente au milieu intermédiaire.

2. Complexe à sondes selon la revendication 1, dans lequel le marqueur de détection a un poids moléculaire de 10 000 ou moins.

3. Complexe à sondes selon la revendication 1 ou 2, dans lequel les marqueurs de détection sont des biotines.

4. Complexe à sondes selon la revendication 1 ou 2, dans lequel les marqueurs de détection sont des haptènes.

5. Complexe à sondes selon la revendication 1 ou 2, dans lequel les marqueurs de détection sont des haptènes, où le haptène est de l'ester d'acridinium.

6. Complexe à sondes selon la revendication 1 ou 2, dans lequel les marqueurs de détection sont des substances fluorescentes ou des substances luminescentes.

7. Complexe à sondes selon la revendication 1 ou 2, dans lequel les marqueurs de détection ont des radioisotopes.

8. Complexe à sondes selon la revendication 1 ou 2, dans lequel le marqueur de détection a des substances fluorescentes et des substances luminescentes, telles que l'isothiocyanate de fluorescéine (FITC) et l'ester d'acridinium.
